# EUROPEAN PATENT SPECIFICATION

(11) **EP 0 372 904 B1**
(45) Date of publication and mention of the grant of the patent: **28.09.1994**
(21) Application number: 89312662.3
(22) Date of filing: 05.12.1989
(51) Int. Cl.: C12N 15/57, C12N 15/54, C12N 15/55, C12N 15/48, C12N 9/50, C12N 9/10, C12N 9/16

(54) **Protease, reverse transcriptase, and endonuclease of retroviruses and method for producing these enzymes**
Protease, reverse Transcriptase und Endonuklease von Retroviren und Verfahren zur Herstellung dieser Enzyme
Protéase, transcriptase inverse et endonucléase des rétrovirus et procédé de production de ces enzymes

(30) Priority: 07.12.1988 JP 309427/88; 06.04.1989 JP 88411/89
(43) Date of publication of application: 13.06.1990
(73) Proprietor: The Research Foundation for Microbial Diseases of Osaka University, Suita-shi Osaka (JP)
(72) Inventor: Saito, Atsusi,, Osaka-fu, (JP); Shinagawa, Hideo,, Suita-shi, Osaka-fu, (JP); Nakata, Atsuo,, Toyonaka-shi, Osaka-fu, (JP)
(74) Representative: Holmes, Michael John

(56) References cited:
- WO-A-87/07296
- GENE, vol. 55, no. 1, 1987; pages 95-103, Amsterdam, NL; S.F.J. LE GRICE et al.: "Expression of biologically active human T-cell lymphotropic virus type III reverse transcriptase in Bacillus subtilis"
- SCIENCE, vol. 236, 17th April 1987, pages 305-307; W.G. FARMERIE et al.: "Expression and processing of the AIDS virus reverse transcriptase in Escherichia coli"
- THE JOURNAL OF BIOLOGICAL CHEMISTRY, vol. 262, no. 26, 15th September 1987, pages 12393-12396; J. HANSEN et al.: "RNase H activity associated with bacterially expressed reverse transcriptase of human T-cell lymphotropic virus III/ lymphadenopathy-associated virus"
- THE EMBO JOURNAL, vol. 6, no. 10, 1987, pages 3133-3137; B. LARDER et al.: "AIDS virus reverse transcriptase defined by high level expression in Escherichia coli"
- THE EMBO JOURNAL, vol. 7, no. 6, June 1988, pages 1785-1791, Heidelberg, DE; J. HANSEN et al.: "Partial purufucation and substrate analysis of bacterially expressed HIV protease by means of monoclonal antibody"
- GENE, vol. 68, 1988, pages 35-42; A. LEUTHARDT et al.: "Biosynthesis and analysis of a genetically engineered HIV-1 reverse transcriptase/endonuclease polyprotein in Escherichia coli"
- JOURNAL OF VIROLOGY, vol. 62, no. 8, August 1988, pages 3053-3058; E.P. LILLEHOJ et al.: "Purification and strucural characterization of the putative gag-pol protease of human immunodeficiency virus"
- THE JOURNAL OF BIOLOGICAL CHEMISTRY, vol. 263, no. 29, 15th October 1988, pages 14617-14620; C.-Z. GIAM et al.: "In Vivo and in Vitro autoprocessing of human immunodeficiency virus protease expressed in Escherichia coli"
- JOURNAL OF VIROLOGY, vol. 62, no. 8, August 1988, pages 2696-2700; M. KOTLER et al.: "Activity of avian retroviral protease expressed in Escherichia coli"
- EMBO Journal, 2, 1791-1794, 1984

## Description

### FIELD OF THE INVENTION

The present invention relates to enzymes coded by retroviral genes, in particular to protease, reverse transcriptase and endonuclease (integrase) enzymes, and to a method for producing them. More particularly, the present invention relates to a method for producing the above-mentioned enzymes in the form of matured or active individual protein molecules rather than as part of a fused protein molecule, by causing expression of at least a kind of gene in which a protease gene is selected as a necessity from the above-mentioned three kinds of enzyme gene group of retrovirus, namely, following four sets of combination; the protease gene alone: the protease and reverse transcriptase genes: the protease and endonuclease genes: the protease, reverse transcriptase and endonuclease genes; by means of the recombinant DNA technique, and at the same time, causing procession of the thus expressed product itself by means of protease within the expressed product. In addition, the present invention also relates to various proteins obtained by this method. The present invention provides such enzymes as protease, reverse transcriptase, endonuclease and gag proteins useful for preparing materials for genetic engineering or retrovirus research, materials for developing pharmacotherapy drugs relating to retrovirus infectious diseases, diagnostic antigens and diagnostic antibodies, as well as for preparing antigens for vaccines.

### PRIOR ART

### [Definition of Retrovirus]

Retrovirus is the generic name of viruses classified as belonging to the retrovirus family, and the features common to these viruses are that they have an envelope, single-stranded RNA genome and reverse transcriptase. These viruses include the spherical shape having a diameter of about 80 to 100nm, composition of two or three molecules of linear (+) stranded RNA genome with molecular weight of about 3 x 10⁶ in the viral particle. More particularly, retrovirus family is further classified into the following three subfamilies, i.e., oncovirus, lentivirus and spumavirus (R.E.F. Matthews Edt. "Classification and Nomenclature of Viruses-Fourth Report of the Internaitonal committee on Taxonomy of Viruses", pp124-pp128, S. Karger [Switzerland], 1982). Known viruses classed as oncovirus, also named RNA tumor viruses, include human T cell leukemia virus, feline leukemia virus, murine sarcoma virus, moloney murine leukemia virus, bovine leukemia virus, hog leukemia virus, avian leukemia virus, avian sarcoma virus, avian myeloblastosis virus, and Rous associated virus. Known viruses classed as lentivirus which are commonly known as viruses causing slow virus infection, include human immunodeficiency viruses types 1 and 2 (hereinafter respectively referred to as "HIV-1" and "HIV-2"), simian immunodeficiency virus, visna virus causing ovine encephalomyelitis, maedi virus causing jaagsiekte, caprine arthritic encephalitis virus, equine infectious anemia virus, and bovine lymphadenitis virus ("Current Topics in AIDS", vol. 1, pp.95-117, John Wiley & Sons, 1987; Advances in Virus Research, vol. 34, p. 189-215, 1988). The viruses classed as spumavirus, also named foamy virus, infect such mammals as humans, monkeys, cattles, and cats. Foamy virus and syncytial virus isolated from these hosts are well known. The term retrovirus as used herein can be taken to include all viruses, known as well as unknown, characterised retroviruses as described above.

### [Present Situation of Fundamental Research Regarding Retroviral Gene]

Retroviruses are important not only from the point of view of the serious and often lethal infectious diseases which they cause in men and other animals, as well as a contagious disease common to them, but they are also useful for understanding of diseases such as sarcoma and for the preparation of material for use in research and genetic engineering. Consequently, as the massive reports about these viruses have been made, the present situation about typical retroviruses is expediently explained in this chapter. As is well known, before 1980 retroviruses had been studied, as a material for the oncogenic mechanism, and from the point of view of clarifying a strange slow virus infectious disease which resulted in incurable diseases. Since the discovery of AIDS in the United States in 1981, comparative studies on various retroviruses have intensively been carried out using the full range of technique in epidemiology, immunology, virology and molecular biology as research materials or experimental models with a view to establishing methods for treatment and prevention of AIDS. A huge volume of useful reports concerning in AIDS has already been accumulated (Advances in Virus Research, vol. 34, pp. 189-215, 1988; Annual Review of Immunology, vol. 6, pp. 139-159, 1988; Microbial Pathogenesis, vol. 5, pp. 149-157, 1988). From among these research reports, an outline regarding HIV genes is described below ("HIV and Other Highly Pathogenic Viruses", pp. 33-41, Academic Press, Inc., 1988; "The Control of Human Retrovirus Gene Expression", pp. 79-89, Cold Spring Harbor Laboratory, 1988; Cytological Engineering, vol. 7 (Suppl. 1), pp. S5-S15, 1988): the viral genome forms a complex with a reverse transcriptase and the structural protein in the core of viral particle, and is present, together with a primer tRNA, in the viral particle; the viral genome comprises about nine different genes, including the basic three major genes encoding the viral particle components essential for virus multiplication, i.e., the gag (group-specific antigen) gene encoding the precursor of the core protein, the pol (polymerase) gene encoding the precursor of three different enzymes, and the env (envelope) gene encoding the precursor of the glycoprotein of the envelope; these genes are arranged from the 5' end to the 3' end in this sequence gag, pol, and env; minutely, gag. pol. vif... and env are arranged adjacent to the respective next ones in this order, and part of the 5' end region of the pol gene overlaps about 240 bases with the 3' end region of the gag gene, with a different reading frame, frame shifting being thought to occur during translation of this overlapping portion, so that translation proceeds through conversion of the termination codon; expression of the entire region of the pol gene having a total length of about 3kb including that overlapping portion leads to production of the above-mentioned enzyme precursor (molecular weight: 160kd) in the form of a fusion protein NH₂ - Gag - protease - reverse transcriptase - integrase - COOH, and then, the thus produced polyprotein cleaved by an existing protease derived from the virus or by the protease activity within the same molecule, and is processed in to the individual mature proteins, i.e., into the Gag proteins and the enzymes protease, reverse transcriptase (p66 and p51) and integrase (p32).

All enzymes mentioned above play important roles in the process of multiplication and maturity of virus or in that of provirus formation, and the following functions have been confirmed or presumed: protease participates in post-translational processing, and core formation or maturity process of viral particle, and the action of protease is highly specific toward viruses from which it is derived; reverse transcriptase functions as an RNA dependent DNA polymerase catalyzing the process of reverse transcription of the genomic RNA into DNA, which is the basic stage of the virus multiplicaiton process, and at the same time, the reverse transcriptase is furthermore known to have the ribonuclease H activity specifically digesting the RNA strand of the RNA-DNA heteroduplex, and the DNA dependent DNA polymerase activity producing double-stranded DNA, and is popularly used as a tool in genetic recombination; integrase is an endonuclease acting on the DNA chain, catalyzing recognition and excision of the part to be integrated into the host chromosome which is of linear or circular virus double-stranded DNA reverse - transcribed from viral genomic RNA through the above-mentioned reverse transcription process and is thus considered to participate in the process of formation of provirus.

### [Present Situation of Applied Research on Retroviral Gene and Problems Involved]

In the area of applied retroviral gene research, active efforts are being made to express the HIV env gene, principally in an attempt to develop a diagnostic reagent or vaccine against AIDS ("Vaccine", pp. 558-567, W. B. Saunders Company, 1988; Science, vol. 18 [No. 12], pp. 110-119, 1988). With regard to research and development in the application area of retroviral gag and pol genes, the following efforts are known in public: for example, a suggestion that the protease gene product is useful as a reagent for the development of an anti-retroviral drug having a high specificity as a therapeutic drug and for the fundamental research on retroviruses (Cytological Engineering, vol. 7 [Suppl. 1], pp. S67-S77, 1988); a method for producing reverse transcriptase using a cell strain established from the hog spleen infected with hog leukemia virus falling under the category of oncovirus (Japanese Patent Provisional Publication No. 59-118,081); a method for producing reverse transcriptase using an Escherichia coli strain transformed with an expression vector carrying the reverse transcriptase gene of avian sarcoma virus falling under the category of oncovirus (U.S. Patent No. 4,663,290); and a method for producing reverse transcriptase comprising preparing a DNA fragment of the reverse transcriptase gene region from pol gene of moloney murine leukemia virus falling under the category of oncovirus, constructing an expression vector carrying said DNA fragment, and then, purifying the product from the culture of the transformant obtained by introducing said expression vector into Escherichia coli (WO 86/06741). Furthermore, the reverse transcriptase enzyme has been used as an antigen in the preparation of a monoclonal antibody for use in the detection of reverse transcriptase derived from avian sarcoma virus (Japanese Patent Provisional Publication No. 61-104,799); as well known currently available, the reverse transcriptase for synthesizing complementary DNA is prepared from avian myeloblastosis virus, and also obtained from moloney murine leukemia virus or Rous associated virus (RAV-2), thus being prepared mainly from oncovirus itself. As is clear from the above description, the prior art concentrated on the expression of the HIV env gene, components of oncovirus, their oncogenic effect and the use of reverse transcriptase gene thereof. As a matter of practical application, difficulties associated with these prior art techniques include the need to protect against biohazards during manufacturing processes, production cost, production yield, and difficulties relating to enzyme activity, substrate specificity, purity, homogeneity and stability. There is, therefore, a need for the development of a safe low-cost mass production system of high-quality products. At the present time, those relating to the usefulness and industrial application of the various retroviral enzymes do not tend to attract much attention, particularly of researchers. In these circumstances therefore, the provision of a new method for the mass production of retroviral enzyme products, i.e., protease, reverse transcriptase and integrase at low cost could be expected to stimulate progress in fundamental research relating to viral infection, and the development of pharmacotherapeutic, diagnostic and preventive drugs, and would thus be of considerable significance.

Hansen et al., in J. Biol. Chem., 262(26): 12393-12396, 1987, describe the cloning of the pol gene of HIV and its expression in E.coli to generate a precursor protein which is cleaved by the action of the HIV protease into the mature reverse transcriptase, endonuclease and protease products. Lillehoj et al., in J. Virol., 62(8): 3053-3058, 1988 and Giam et al., in J. Biol. Chem., 263(29): 14617-14620, 1988 describe the expression of HIV pol and protease regions respectively, as fusion proteins with part of the LacZ protein. Leuthardt et al., in Gene, 68: 35-42, 1988 explain how autoproteolytic cleavage is believed to operate in the pol open reading frame of HIV-1 to yield mature protease, reverse transcriptase and endonuclease products. Similarly Kotler et al., in J. Virol., 62(8): 2696-2700, 1988 disclose the processing of an avian retroviral gag protein by co-expression with a protease gene.

### OBJECT OF THE INVENTION

In attempts to overcome the above-mentioned difficulties, we have studied energetically, and as a result achieved a method for mass-producing retroviral enzyme products i.e., enzymes such as protease, reverse transcriptase and integrase, safely in terms of biohazard, at a stable and high production yield, with a low cost. This achievement is due to success in causing connection of enzyme gene cDNA fragment of the said virus prepared in the manner of containing a retroviral protease gene as a necessity with inducible gene with expressing ability by matching those translating frames by the full utilization of the recombinant DNA technology, raising expression of the enzyme gene products, and processing the gene product itself by the expressed protease. We found it possible to produce stably in large quantities the above-mentioned enzymes coded by that cDNA, not as fusion protein, but individual mature proteins having a specific activity in the culture, by preparing transformant obtained through introduction of an expression vector carrying the above-mentioned gene cDNA, and applying the two-stage culturing method as described later for culture of the said transformant. We found also that such a processing was due to the specific activity of protease accounting for part of the fusion protein which are expression products of the above-mentioned gene, more particularly, that the processing was a phenomenon unique to the retroviral protease. In addition, we have found that these enzymes have very high purity and homogeneity as a result of improve of the mass production and purification processes, and particularly when retroviral genes expressed resulting enzymes have an activity with a very high substrate specificity unique to retroviruses. The present invention was achieved on the basis of these findings.

According to the present invention, there is provided a method for preparing one or more retroviral proteins, said method comprising the steps of:
(i) constructing an expression vector comprising a cDNA sequence corresponding to one or more retroviral genes encoding at least one retroviral protein, linked in matching reading frame to a sequence corresponding to the entire lacZ gene or to a lacZ gene lacking the nucleotides coding for one or both of the two C-terminal amino acids , with the proviso that at least one of said retroviral genes encodes a protease;
(ii) introducing the said expression vector into cells of E.coli strain UT481 (FERM BP-2417);
(iii) culturing the transformed cells under conditions whereby the said genes are expressed as a fusion-protein which is subsequently processed in situ by the action of the said protease to form separate mature protein molecules, wherein where at least one other retroviral protein is expressed in addition to the protease, the transformed cells are cultured at about 25°C following induction.
Retroviral enzymes such as protease, reverse transcriptase and integrase produced by such a method are useful as tools in genetic engineering for the dissociation and cleavage of viral components, synthesis of complementary DNA, preparation of proviruses through integration of viral genomes into the host cell and transformation of the host cell; they may also be used
as tools in protein engineering useful for the functional and structural analysis of protein;
as virological tools for fundamental and clinical studies useful for the clarification of multiplication mechanism of viruses and for the development of antiviral drugs exerting specific effects on retroviruses;
as diagnostic antigens and for the preparation of diagnostic antibodies to detect retroviral infections, or as antigens for the preparation of immunoglobulin for use in therapy or for the preparation of vaccine for the prevention of secondary infection of retroviruses; and in addition, the above-mentioned enzymes may be useful as materials using functions and features of protease, reverse transcriptase and integrase known at present and to be clarified in the future.

### BRIEF DESCRIPTION OF THE DRAWINGS

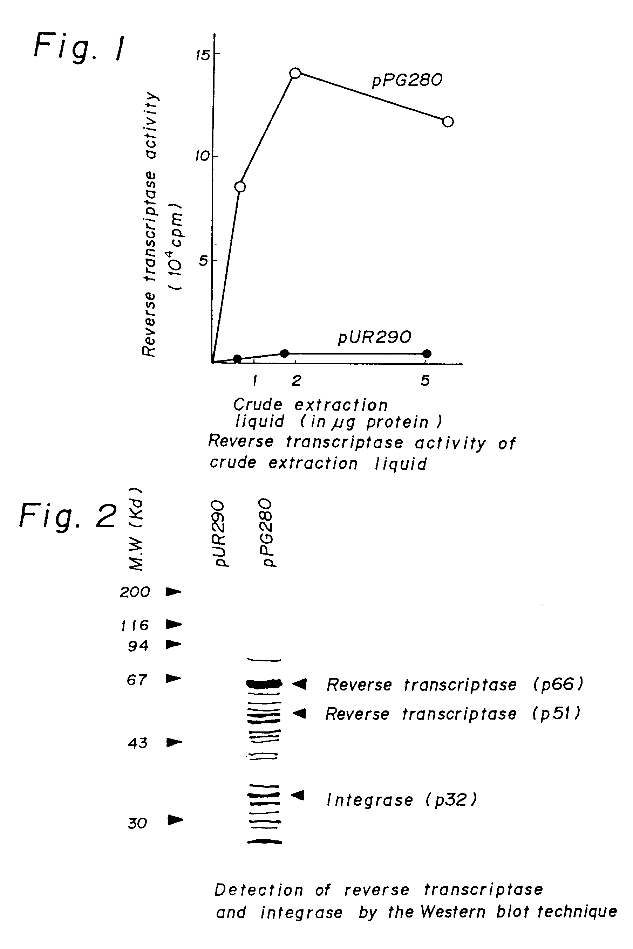

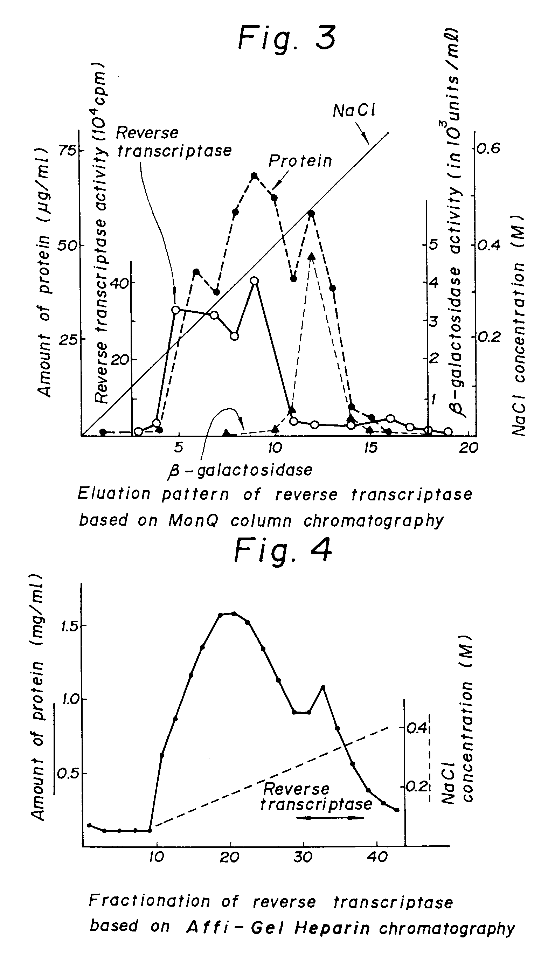

Fig. 1 is a graph illustrating titers of reverse transcriptase activity of the crude extracts of Escherichia coli transformed with the plasmid pPG280 carrying the HIV pol gene, and Escherichia coli transformed with the vector pUR290 not having the HIV pol gene; Fig. 2 is a graph illustrating the result of Western blot analysis using human serum obtained from HIV carriers, of crude extracts of Escherichia coli transformed with the plasmid pPG280 carrying the HIV pol gene and the vector pUR290 not carrying the HIV pol gene; Fig. 3 is a graph illustrating the elution profile of reverse transcriptase derived from Escherichia coli crude extracts on an anion exchange column; and Fig. 4 is a graph illustrating separation of reverse transcriptase by Affi-Gel Heparin chromatography.

### DETAILED DESCRIPTION OF THE INVENTION

The present invention has the following construction:
(I) Selection of retroviral enzyme genes and preparation of DNA fragments: various enzymes of retroviruses founded on the above-mentioned "Definition", such as protease, reverse transcriptase and integrase, can be used in terms of the enzyme gene of retrovirus. These genes are used as four sets of combination containing a protease gene as a necessity, as described in "FIELD OF THE INVENTION". And, in the case of gene expression by means of the recombinant DNA technique, the above-mentioned various genes are used by being converted into a complementary DNA, for the retroviral genome is an RNA. Such cDNA can be prepared by cloning a proviral genome or the integrated genomic DNA. And or, using a genomic RNA extracted from the viral particle, that cDNA also can be prepared by being selected from the cDNA library which has been made in accordance with conventional method. However, these preparations are not necessarily easy from the point of view of avoiding infection under direct operation of a retrovirus having a high degree of hazard. Therefore, in order to avoid biohazard being due to such virus and to save labor in the above-mentioned preparation processes, it is recommend to use a known and cloned retroviral genome. As is seen in the general description cited above, the cloning of various retroviral genomes, the preparation of restriction enzyme maps and the determination of nucleotide sequences have already been reported by researchers throughout the world, and utilization of their achievement may be desirable because of their security and convenience. The available clone include, for example, a plasmid SRA2 (Journal of Virology, vol. 36, pp. 50 - 61, 1980) which carries the avian sarcoma virus genome, HIV-1 provirus genome clones, i.e., plasmids pNL3-1, pNL3-2 and pNL4-3 (Journal of Virology, vol. 59 [No. 3], pp. 284-291, 1986), and plasmid pNLH402 of E. coli strains UT481/pNLH402 (Microbiology Research Inst. Registration No. 10436). cDNA fragments can be prepared from the these plasmids by conventional methods, for example, by excising the DNA from the required region of the above-mentioned plasmid clones by means of a restriction enzyme and purifying the resultant product through phenol extraction, chloroform treatment or ethanol precipitation. The restriction enzyme used for excision of the DNA fragments may appropriately be selected by reference to the genomic DNA clone restriction enzyme map. Thus for example to excise DNA fragments from the entire gene region of the above-mentioned pNLH402, the restriction enzyme HindIII (Journal of Virology, vol. 59, pp. 284-291, 1986) may be employed.
(II) Construction of expression vector, and preparation of transformant introduced with the vector: expression vector is constructed by linkage the retroviral genomic cDNA fragment prepared as described above by a conventional method such as that using T4DNA ligase. Any of the following vectors may be used for expression purposes; those conventionally known or commercially available, for example, plasmid vectors of the pSN508 series of the enteric bacteria family (U.S. Patent No. 4,703,005), plasmid vector pJM105 (Japanese Patent Provisional Publication No. 62-286,930), vectors of pBH103 series (Japanese Patent Provisional Publicaiton No. 63-22,098) of yeast, attenuated varicella virus vector (Japanese Patent Provisional Publication No. 53-41,202), attenuated Marek's disease virus vector (Journal of the Japan Veterinary Society, vol. 27, pp. 20-24, 1984; and Gan Monograph on Cancer Research, vol. 10, 1971), Escherichia coli plasmid vector pUR290 series (EMBO Journal, vol. 2 [No. 10], pp. 1791-1794, 1983), and pSN5182 (Journal of Bacteriology, vol. 157, pp. 909-917, 1984). What is important in the construction of the expression vector is to link the above-mentioned enzyme gene in matching reading frame with a gene which is capable of being highly expressed. Thus for example, when using pUR290 referred to above, the pol gene should preferably be inserted downstream of lacZ gene of the plasmid, or in the case of pSN5182, downstream of the plasmid pstS gene. Furthermore, as carrying that gene, attention should be given to matching the codon reading frames among the genes so as to ensure smooth progress of translation. For example, when the cDNA of such as HIV-1, HIV-2, simian immunodeficiency virus and moloney murine leukemia virus are inserted, the reading frame of the pol gene is linked so as to match with those of genes with high expressing ability, because of the protease of such viruses as described above are encoded in the pol gene region. On the other hand, a protease of avian sarcoma virus is encoded in the gag gene region having a different reading frame from the pol gene, and the protease gene of human T-cell leukemia virus or bovine leukemia virus has yet another reading frame differing from those of both the pol and gag genes. In these cases, care is needed to match the reading frames of all the genes, i.e., the retroviral genes e.g. protease gene, pol gene and the gene with high expressing ability, in order to ensures signigicant expression of the retrovial genes. Matching of the reading frames above can be accomplished using conventional techniques employing enzymes such as restriction enzyme, nuclease Bal31 and mung bean nuclease. The optimum recipient cell used for the purpose of obtaining a transformant through introducing of the thus constructed expression vector should be selected from among host cells allowing multiplication and expression of that expression vector, and at the same time, from these host cells, a cell permitting easy introduction of the expression vector built as mentioned above and certain detection should be strictly selected and used. When using the above-mentioned pSN series plasmids as the expression vector for example, it is desirable to use Escherichia coli C75 strains (Microbiology Research Inst. Registration No. 10191) as the host cells, which transforms in appearance from an alkaline phosphatase non-productive bacteria into a productive one by the introduction of that vector, as the recipient bacteria, and when using pUR290 series, it is able to employ Escherichia coli UT431 (Journal of Bacteriology, vol. 163, pp. 376-284, 1985) which permits selection of a transformant introduced with this vector, with ampicillin resistance as the marker. Introduction of the expression vector into such a recipient cell may be accomplished by a conventional method such as the calcium chloride method (Journal of Molecular Biology, vol. 53, pp. 154-162, 1970). The transformant introduced the enzyme gene expression vector as described above is selected by the above-mentioned marker from the positive colony. Then, after extracting the expression vector DNA through selection from the colony of transformant,it is digested with a restriction enzyme, and the resultant DNA fragments are subjected to agarose gel electrophoresis. Subsequently, the size of the inserted DNA fragment can be measured, and simultaneously, the colony in which the presence of DNA fragment of that gene has been confirmed is adopted as the transformant clone of retroviral enzyme gene expression. For example, when insertion covers the entire pol gene region prepared from pNLH402 into the above-mentioned expression vector pUR290, EcoRI fragment of about 4kb DNA can be detected.
(III) Confirmation of retroviral enzyme genes expression by the transformant clone and mass production of various enzymes by culture of said transformant: Confirmation of the enzyme gene expression by the transformant clones can be accomplished, for example, by analyzing the crude extraction liquid of the products of that clone by the use of the Western blot technique. The crude extract can be prepared, for example, by culturing and inducing the transformant in a conventional culture medium, collecting cells by low-speed centrifugation, treating the collected cells with sodium dodecyl sulfate and 2-mercaptoethanol, subjecting them to high-speed centrifugation, and collecting the supernatant liquid. The Western blot technique may be carried out in accordance with the conventional procedure useing various commercially available materials in the following steps: subjecting the above-mentioned crude extracs to polyacrylamide gel electrophoresis; transferring the separated protein onto a nitrocellulose membrane by the use of a transblotting apparatus, and immersing the membrane into gelatin solution for blocking. The subsequent steps include, when the specimen on the membrane is an HIV pol gene product, for example: causing a primary reaction with human serum of HIV carrier: causing a secondary reaction with peroxidase-conjugated anti-human IgG antibody after washing; causing coloring with hydrogen peroxide solution and a chromogenic agent after washing and detecting a band specifically reacting with the human serum of HIV carrier, thereby confirming expression of the pol gene by the above-mentioned clone. In the case of the specimen being the gene product originating from a retrovirus other than HIV, an human serum of HIV carrier is not employed, but an appropriate retroviral antiserum is used for the primary reaction, and an antibody to human or animal IgG is used for the secondary reaction.
   Mass production of the various enzymes such as protease, reverse transcriptase and integrase through culture of transformant for which enzyme expression has been confirmed is conducted as follows: transformant of Escherichia coli is cultured in LB medium at a temperature of from 30 to 40°C for from 12 to 35 hours until a bacteria concentration of from 10⁹ to 10¹⁰ cells/ml is reached to prepare seeds for large-scale culture of that transformant; then, inoculating such seeds into fresh medium prepared, and conducting two-stage culture consisting of a pre-culture and an after-culture. The pre-culture is carried out for the purpose of multiplying seed cells and amplifying the expression vector at a temperature of from 10 to 40°C for from 1 to 24 hours, or more preferably at a temperature of from 15 to 37°C for from 2 to 12 hours. The pre-culture is discontinued, in the case of Escherichia coli, with a concentration of bacteria in culture, i.e., a turbidity of the culture liquid of OD600ₙₘ = 0.4 to 0.7 as the standard. Subsequently, upon completion of this pre-culture, conditions for the induction-culture should be set with the most careful attention and contrivance so that transcription and translation of enzyme gene linked to expression vector and the gene product after translation is properly modified, and to ensure processing to achieve individual and single matured proteins having an activity, as well as to avoid that the enzyme gene product after translation is unorderly decomposed by proteolytic enzyme originating from the host cells and thus loses its activity. The culture induced should preferably be carried out at a temperature of from 10 to 30°C for from 1 to 40 hours, or more preferably, at a temperature of from 15 to 28°C for from 3 to 35 hours. Considering the property of the expression vector used, expression may be induced or accelerated, for example, by causing starvation of phosphate ion in the medium at the start of the induction-culture or by adding and mixing an inducer into the medium. Applicaiton of the above-mentioned two-stage culture permits production of various enzymes of retrovirus such as protease, reverse transcriptase and integrase , not in the form of fusion proteins, but as independent active proteins, i.e., as individual and single mature proteins usually at a high yield of from 1 to 10mg per liter of medium.
(IV) Purification of various retroviral enzymes such as protease, reverse transcriptase and integrase which are massproduced by means of expression vector.: This step can be accomplished by any combination of conventional methods, including, for example; extraction of cultured product of transformant through the use of precipitants, centrifugation or filtration; preparation of crude extracs through breakage or crushing of the transformed cells by the application of ultrasonic treatment, high pressure treatment or a homogenizer; purification through adsorption-elution treatment by means of silica or activated charcoal, salting-out, or precipitaion by means of organic solvents; high-grade purificaiton by means of ultracentrifugation, column chromatography or electrophoresis; or a method for purifying a gene product through fractionation by density-gradient centrifugation following adsorption-elution with silica and activated charcoal (Japanese Patent Provisional Publication No. 63-297).

The enzymes such as protease, reverse transcriptase and integrase available by the method of the present invention may be provided in the form of liquid, dried powder or adsorbed onto filter paper or a membrane, and enclosed in an ampul, a vial or other small container. In the dried powder, the enzyme may be used in a necessary amount after dissolving in distilled water to the volume before using. When it is adsorbed onto filter paper or membrane, it should be used after wetting with a solution as prescribed in the instruction.

The method of the present invention is described below in more detail with reference to examples. The present invention is not limited to the examples described below.

### (Experiment 1)

Measurement of activity of reverse transcriptase: a reaction mixture is made up comprising 50mM tris-HC1(pH8.3), 50mM potassium chloride, 10mM magnesium chloride, 3mM dithiothreitol, 0.1W/V% Nonidet P-40(made by Shell Oil [U. S. A.]). 20»g/ml (rA)ₙ(dT)₁₂₋₁₈ (Pharmacia [Sweden]), 0.5mM dTTP (deoxy thymidine triphosphate), and 1»Ci [³H] dTTP (deoxy thymidine triphosphate). This reaction mixture was added with specimen in an amount of 5»l into a total volume of 50» , and the mixture was incubated at 37°C for ten minutes. Then the mixture is immediately cooled on ice, and filtered through a filter paper DE81 (made by Wattman[England]). The filter is washed well with 5% sodium phosphate solution, and then with enthanol after water rinsing. After drying, radioactivity is measured by means of a liquid scintillation counter.

### (Example 1)

Construction of an expression vector carrying the pol gene of lentivirus: 5»g of plasmid pNL4-3 DNA (Journal of Virology, 59(2): 284-291, 1986) carrying the HIV proviral genome DNA was added to 5»l HindIII, 20»l 5 x RM (50mM tris-HCl [pH 7.5], 35mM MgCl₂, 300mM NaCl), diluted with distilled water to a total volume of 100»l, and after incubation at 37°C for an hour, extraction of the solution was carried out with phenol saturated with TE(10mM tris-HCl [pH 7.5], 1mM EDTA). The water layer was treated with chloroform before ethanol precipitation. To the mixture of 1»l of the solution prepared by dissolving the precipitation into 10»l TE, 0.1ug (1»l) of plasmid pHSG398 DNA cleaved by HindIII and treated with alkaline phosphatase, and 2»l of 10 x ligation buffer (660mM tris-HCl [pH7.6], 66mM MgCl₂, 100mM DTT and 1mM ATP), 1»l T4DNA ligase was further added and the total volume was brought up to 20»l with distilled water. Then, incubation was applied at 15°C for 12 hours. Subsequently, Escherichia coli strain JM103 was transformed with this reation liquid in accordance with the calcium chloride method (Journal of Molecular Biology), 53: 154,1970), and chloramphenicol resistant colonies were selected on an LB medium plate (1w/v% Bacto-trypton, 0.5W/V% Bacto-yeast extract, 1W/V% Nacl and 1.5W/V% agar) containing 20»g/ml chloramphenicol. Plasmid DNA was extracted from the chloramphenicol resistant clone by a conventional method, and clone pNLH402 was obtained by selecting a clone containig about 4.0kb fragments originating from plasmid pNL4-3 DNA through HindIII excision.

HindIII in an amount of 5»l and 5 x RM in an amount of 10»l were added to 5»l (5»g) of plasmid pNLH402 DNA, and the mixture was diluted with distilled water to a total volume of 50»l. The mixture was incubated at 37°C for an hour, and after phenol extraction and chloroform treatment, the mixture was subjected to ethanol precipitation. The resulting precipitate was added to 10»l of 5 x RM and 5»l of BglII and was diluted with distilled water to a total volume of 50»l, whereby it was completely dissolved. The mixture was incubated again at 37°C for an hour, and after phenol extraction and chloroform treatment, the resulting product was subjected to ethanol precipitation. The thus obtained DNA was dissolved into 10»l of TE.

At the same time, 5»l of HindIII and 10»l of 5 x RM were added to 5»g of expression vector pUR280 DNA (The EMBO Journal, 2(2): 1791-1794, 1983). The mixture, diluted with distilled water to 50»l, was incubated at 37°C for an hour, and after phenol extraction, chloroform treatment and ethanol precipitation, 10»l of 5 x RM (NaCl concentration: 500mM) and 5»l of BamHI were added to it. 35»l of distilled water were further added so as to cause complete dissolution of the precipitate, and the solution was then incubated at 37°C for an hour. After phenol extraction and chloroform treatment, DNA precipitated with ethanol was dissolved into 10»l of TE.

Then, pUR290 DNA (1»l) digested with HindIII and BamHI was mixed with pNLH402 DNA (1»l) digested with HindIII and BglII and 2»l of 10 x ligation buffer and 1»l of T4DNA ligase were added. A total volume of 20»l was achieved with distilled water, and reaction was caused at 15°C for 12 hours. Escherichia coli strain UT481 (Journal of Bacteriology, 163: 376-387, 1985) was transformed with the reaction liquid in accordance with the above-mentioned calcium chloride method. Ampicillin resistant colonies were selected on an LB medium plate containing 20»g/ml ampicillin, and furthermore, a clone containing fragments of about 3.8kb originating from pNL4-3 was selected by measuring the size of the inserted fragment by EcoRI cleavage. Clone UT481/pPG280 was thus obtained. More specifically, in this clone the approximately 3.8kb HIV pol gene region is considered to be ligated to the 3' end of lacZ gene of plasmid pUR290, and the lacZ and pol gene product is initially expressed as a fusion protein (about 230kd), the various separate enzymes being produced after processing.

### (Example 2)

Production of lentiviral protease, reverse transcriptase and integrase enzymes by culture of transformed cells: After culturing transformed cell clone UT481/pPG280 at 37°C for 18 hours in an LB medium containing 20»g/ml ampicillin (1W/V% Bactotrypton, 0.5W/V% Bacto-yeast extract and 1W/V% NaCl), the resultant cells were added to fresh LB medium containing 20»g/ml ampicillin at 1:100 dilution and the the pre-culture was carried out. When the OD600ₙₘ of the medium reached 0.5, 1mM IPTG (Isopropyl-β-D-thiogalacto pyranoside, made by Sigma [U. S. A.]) was added, and culture was continued at 25°C for 18 hours. Bacteria were collected by centrifugation (5,000rpm for five minutes) and suspended in 1/25 volume of 40mM tris-HCl (pH 8.0) (0.1mM EDTA, 5mM MgCl₂, 0.1W/V% Triton X-100 and 10mM 2-mercaptoethanol). After ultrasonic treatment (five 30-second bursts, 19.5kHz, 300W), the supernatant liquid was separated by centrifugation (19,000rpm, 60 minutes). To confirm the presence of HIV pol gene product in this crude extraction liquid, the activity of the reverse transcriptase in the crude extraction liquid was measured. The result is shown in Fig. 1. The expected significant activity of the reverse transcriptase was observed. Analysis by the Western blot technique was also carried out: 4W/V% sodium dodecyl sulfate (SDS) and 1W/V% 2-mercaptoethanol were added to the collected bacteria. After boiling for five minutes and centrifugation (10,000 rpm for five minitues), the supernatant liquid was electrophoresed on a 0.1W/V% SDS - 10W/V% polyacrylamide gel. After blotting onto a nitrocellulose membrane (made by S&S [West Germany]) by means of transblotting apparatus (made by BioRad [U. S. A.]), the membrane was immersed in 3W/V% gelatin solution in accordance with the conventional blocking method. Then, as a primary reaction the membrane was incubated with human serum obtained from a HIV carrier, and after washing, as a secondary reaction was caused with peroxidase marker conjugated anti-human IgG antibody (made by BioRad). Finally, after washing, the membrane was immersed in a chromogenic liquid prepared by adding 0.4ml of DAB (3,3'-diaminobenzidine tetrahydrochloride) and 15»l of 30W/V% hydrogen peroxide solution to 50ml of TBS (20mM tris-HCl [pH 7.4], 500mM NaCl), to cause color formation, at room temperature for 15 minutes, and was then washed with distilled water. The result is shown in Fig. 2. While no specific band reacting with human HIV carrier serum was observed in the crude extraction liquid of the transformed cell UT481/pUR290 based on the vector pUR290 not carrying a HIV pol gene, bands of reverse transcriptase having molecular weight of 66kd and 51kd, integrase of 32kd, and protease of 12kd, i.e. the HIV pol gene products, were observed in the extraction liquid of transformed cells of strain UT481/pPG280. Cleavage of the reverse transcriptase from β-galactosidase is easily known from the result of column chromatography with anion exchanger MonoQ (made by Pharmacia [Sweden]) as shown in Fig. 3. More particularly, the reverse transcriptase activity can be found in a fraction completely separated from β-galactosidase activity. This suggests that, although HIV pol gene products are produced as fusion proteins with β-galactosidase, protease, reverse transcriptase, and integrase regions of that fusion protein are specifically separated by the action of the protease which is itself a pol gene product, and accumulated in the cell.

### (Example 3)

Construction of a vector to enable the production of large amounts of lentiviral protease; 5»l of HindIII and 10»l of 5 x RM were added to 5»g of DNA of the pol gene expression plasmid pPG280 prepared in Example 1, and the mixture was diluted with distilled water to a total volume of 100»l. The mixture was incubated at 37°C for an hour, and after phenol extraction and chloroform treatment, the mixture was subjected to ethanol precipitation. The resultant precipitation was added to 5»l of 5 x RM (-NaCl) and 5»l of BalI and was diluted with distilled water to a total volume of 50»l, whereby the precipitate was sufficiently dissolved. The mixture was incubated again at 37°C for an hour, and after phenol extraction and chloroform treatment, the resulting product was subjected to ethanol precipitation. The resulting precipitation was added to 5»l of 10 x polymerase buffer (670mM Tris HCl [pH 8.8], 67mM MgCl₂, 166mM (NH₄)₂SO₄, 100mM 2-mercaptoethanol and 67»M EDTA), 5»l of 10 x dNTP solution (each 3.3mM of dATP, dGTP, dTTP, and dCTP) and 1»l T4 DNA polimelase and was diluted with distilled water to a total volume of 50»l, whereby it was sufficiently dissolved. The mixture was incubated at 37°C for 15 minutes, and after phenol extraction and chloroform treatment, the resulting product was subjected to ethanol precipitation. To the mixture of 1»l of the solution prepared by dissolving the resultant precipitation into 10»l of TE and 2»l of 10 x ligation buffer, 1»l of T4 DNA ligase was further added and total volume was brought up to 20»l with distilled water. The mixture was further incubated at 15°C for 12 hours. Escherichia coli strain UT481 was transformed with this reaction liquid in accordance with the above-mentioned calcium chloride method. Ampicillin resistant colonies were selected on an LB medium plate containing 20»g/ml ampicillin, and furthermore, a clone containing 0.55kb fragment originating from pNL4-3 was selected by measuring the size of the inserted frangment using EcoRI digestion. Clone UT481/pLB550-3 was thus obtained.

### (Example 4)

Mass production of lentiviral protease by transformed cells: After culturing transformant clone UT481/pLB550-3 at 37°C for 18 hours in LB medium (containing 20»g/ml ampicillin), the resulting cells were added to fresh LB medium (containing 20»g/ml ampicillin) at 1:100 dilution and the pre-culture was carried out at 37°C. When the OD600ₙₘ of the medium reached 0.5, 1mM IPTG (Isopropyl β-D-thioglacto pyranoside, Sigma [U. S. A.]) was added, and culture was continued at 37°C for 6 hours. Bacteria were collected by centrifugation (5,000rpm for five minutes), and 4W/V% sodium dodecyl sulfate (SDS) and 1W/V% 2-mercaptoethanol were added. After boiling for five minutes and centrifugation (10,000rpm for five minutes), the supernatant liquid was electrophoresed on a 0.1W/V% SDS - 15W/V% polyacrylamide gel. Subsequently, the collected bacteria were analyzed by means of the Western blot technique described in Example 2. While no specific band reacting with human HIV carrier was observed in the crude extracts of UT481/pUR290, bands of 12kb protease serum was observed in the extracts liquid of UT481/pLB550-3. Especially, pLB550-3 produced an amount of protease several times as much as pPG280. In this clone, 0.55kb HIV pol gene is considered to be ligated to the 3' end of lacZ gene of plasmid pUR290, and the lacZ - pol gene product is estimated to be produced as a fusion protein with molecular weight of about 140kb, and a protease of about 12kb being produced after processing.

### (Example 5)

Construction of an expression vector carrying oncoviral protease and pol gene; 5»g of plasmid pSRA2 DNA carrying Rous sarcoma virus cDNA (Journal of Virology, 36, pp50-61, 1980) was added with 5»l of BamHl and 20»l of 5 x RM, and was diluted with distilled water to a total volume of 100»l, which was then incubated at 37°C for an hour. After this reaction, the mixture was electrophoresed on a 1W/V% agarose gel having a low melting point, and the gel portion containing a 1.8kb DNA fragment was digested. Then, after phenol extraction and chloroform treatment, the resulting product was subjected to ethanol precipitation. To the mixture of 1»l of the solution prepared by dissolving the precipitation into 10»l of TE, 0.1»g (1»l) of plasmid pUR291 DNA cleavaged by BamH1 and treated with alkaline phosphatase, and 2»l of 10 x ligation buffer, 1»l of T4 DNA ligase was further added and the total volume was brought up to 20»l with distilled water. The reaction mixture was incubated at 15°C for 12 hours. Subsequently, Escherichia coli strain UT481 was transformed with this reaction mixture in accordance with the calcium chloride method, and ampicillin resistant colonies were selected on an LB medium plate containing 20»g/ml ampicillin. Plasmid DNA was extracted from the ampicillin resistant clone using a conventional method, and a clone pSR281 was obtained by selecting a clone containing a 1.8kb fragment originating from plasmid pSRA2 and producing a LacZ-Gag fusion protein.

5»g of plasmid pSRA2 DNA was added to 5»l of PstI and 20»l of 5 x RM (750mM NaCl), and was diluted with distilled water to a total volume of 100»l, which was then incubated at 37°C for an hour. After this reaction, the mixture was electrophoresed on a 1W/V% agarose gel having a low melting point, a 1.8kb DNA fragment was digested. Then, after phenol extraction and chloroform treatment, the resulting product was subjected to ethanol precipitation and dissolved to 10»l of TE. Similarly, the double-stranded phage DNA of M13mp18 was cleaved by PstI and treated with alkaline phosphatase. A 1»l (0.1»g) of this DNA was added to 1»l of 3.1kb DNA fragment mentioned above, 2»l of 10 x ligation buffer and 1»l of T4 DNA ligase, and was diluted with distilled water to total volume of 100»l, which was then incubated at 15°C for 12 hours. Subsequently, the recombinant phage DNA was used to transfect Escherichia coli strain TG1 following the calcium chloride method, and a plaque was formed on a 2YT medium plate (1.6W/V% Bacto-trypton, 1W/V% Bacto-yeast extract, 0.5W/V% NaCl and 1.5W/V% Bacto-agar) containing an X-gal (5-brom-4-chloro-3-indolyl-β-D-galactopyranoside, Sigma [U. S. A.]).

Next, the TG1 strain was propagated in a 2YT medium (1.6W/V% Bacto-trypton, 1W/V% Bacto-yeast extract, and 0.5W/V% NaCl) until the OD600ₙₘ of the medium reached 0.3, and some of the achromatic clone of the resultant plaque were inoculated. Each single-and double-stranded DNA was prepared in accordance with a conventional method after continuing to incubate for several hours. A clone M13sr31 which contains
a 3.1kb fragment originating from pSRA2 was selected by digesting the obtained double-stranded DNA with PstI and BamHI. The 3.1kb fragment originating from pSRA2 encodes 3' end of gag gene, the termination codon TAG, and the pol gene. The insertion of one base before the termination codon results in the expression of a gag-pol fusion gene having matching translating frames. Thus by using an in vitro mutagenesis kit (made by Amersham[England]), a clone M13sr32 was obtained, containing the sequence ATAG obtained by inserting one base before the termination codon TAG on the M13sr31.

5»g of double-stranded DNA of M13sr32 was added to 5»l of Pst1 and 20»l of 5 x RM, and was dilluted with distilled water to total volume of 100»l, which was then incubated at 37°C for an hour. After this reaction, the mixture was electrophoresed on a 1W/V% agarose gel having a low melting point, and a gel containing a 3.1kb DNA fragment was digested. Then, after phenol extraction and chloroform treatment, the resulting product was subjected to ethanol precipitation. To the mixture of 1»l of the solution prepared by dissolving the precipitation into 10»l of TE, 1»l (0.1»g) of plasmid pSR281 DNA digested by PstI and treated with alkaline phosphatase, and 2»l of 10 x ligation buffer, 1»l of T4 DNA ligase were further added and the total volume was brought up to 20»l with distilled water. Then, the mixture was incubated at 15°C for 12 hours. Subsequently, Escherichia coli UT481 strain was transformed with this reaction mixture in accordance with the calcium chloride method, and ampicillin resistant colonies were selected on an LB medium plate containing 20»g/ml amplicillin. Plasmid DNA was extracted from the ampicillin resistant clone by a conventional method, and the presence and direction of the 3.1kb frangment originating from M13sr32 were confirmed by digesting the plasmid by PstI and BamHI, and then a clone UT481/pSR271 which was assumed to express protease and pol gene products was obtained.

More especially, among the 1.8kb region originating from pSRA2, which was contained in a pSR281, a 1.3kb region overlapping with 3.1kb fragment originating from M13sr32 was removed when pSR281 was cleaved by PstI.

### (Example 6)

Production of oncoviral protease, reverse transcriptase and integrase enzymes by culture of transformed cells: After culturing transformant clone UT481/pSR271 at 37°C for 18 hours in an LB medium (containing 20»g/ml ampicillin), the resultant cells were added to fresh LB medium (containing 20»g/ml ampicillin) at 1:100 dilution and the pre-culture was carried out. When the OD600ₙₘ of the medium reached 0.5, 1mM IPTG was added, and culture was continued at 25°C for 18 hours. Bacteria were collected by centrifugation (5,000rpm for five minutes) and suspended in 1/25 volume of 40mM tris-HCl (pH 8.0) (0.1mM EDTA, 5mM MgCl₂, 0.1W/V% Triton X-100 and 10mM 2-mercaptoethnol). After ultrasonic treatment (five 30-second bursts, 19.5kHz, 300W), the supernatant was separated by centrifugation (19,000rpm, 60 minutes). To confirm the presence of RSV gene product in this crude extraction liquid, the activity of the reverse transcriptase in the crude extraction liquid was measured. The expected significant activity of the reverse transcriptase was observed. Analysis by the Western blot technique was also carried out: 4W/V% sodium dodecyl sulfate (SDS) and 1W/V% 2-mercaptoethanol were added to the collected bacteria. After boiling for five minutes and centrifugation (10,000rpm for five minutes), the supernatant was electrophoresed on a 0.1W/V% SDS - 15W/V% polyacrylamide gel. After blotting onto a nitrocellulose membrane (made by S&S [West Germany]) using transblotting apparatus (made by BioRad [U. S. A]), the membrane was immersed in 3W/V% gelatin solution in accordance with the conventional blocking method. Then, as a rimary reaction the membrane was incubated with anti-RSV rabbit serum, and after washing, as a secondary reaction was incubated with peroxidase marker conjugated anti-rabbit IgG antibody (made by BioRad). Finally, after washing, the membrane was immersed in a chromogenic liquid prepared by adding 0.4ml of DAB (3.3'-diaminobenzidine tetrahydrochloride) and 15»l of 30W/V% hydrogen peroxide solution to 50ml of TBS (20mM tris-HCl [pH 7.4], 500mM Nacl), to cause color formation, at room temperature for 15 minutes, and was then washed with distilled water. While no specific band reacting with anti-RSV rabbit serum was observed in the crude extraction liquid of the transformed cell UT481/pUR290 based on the vector pUR290 not having RSV gene, bands of RSV reverse transcriptase were observed in the extraction liquid of UT481/pSR271. Although RSV protease and the pol gene product are produced as fusion protein with β-galactosidase, proteases and reverse transcriptase regions are specifically separated by the action of the protease which is itself a gag gene product, and are estimated to be accumulated in the cell. In the clone UT481/ pSR271, the 3.6kb Rous sarcoma virus gag and pol gene region is considered to be ligated to the 3' end of lacZ gene of plasmid pUR291, and it is suggested that the lacZ, gag and pol gene products are expressed as a fusion protein (about 230kb), which is then processed to liberate the enzymes e.g. protease (P15), reverse transcriptase (P92, P65) and integrase (P32).

### (Example 7)

Extraction of reverse transcriptase: As mentioned above in Example 2, transformed Escherichia coli clone UT481/pPG280 was cultured in 91 LB medium (containing 20»g/ml ampicillin) at 25°C, and when the culture reached an OD600ₙₘ of 0.5, 1mM IPTG was added. Culture was further continued for another 24 hours, and after collection, the cells were suspended in 120ml of 40mM tris-HCl (pH 8.0) (containing 0.1mM EDTA, 5mM MgCl₂, 0.1W/V% Triton X-100 and 10mM 2- mercaptoethanol) buffer. Bacterial cells were crushed by ultrasonic treatment and subjected to centrifugation (19,000 rpm for 60 minutes), and the supernatant was separated as the crude extraction liquid.

### (Example 8)

Purification of reverse transcriptase: Polymine P (made by BRL[U. S. A]) was added in an amount of 0.1W/V% to the crude extraction liquid, which was then stirred at 4°C for 30 minutes and centrifugated (16,000rpm for 20 minutes).

Ammonium sulfate was added to the supernatant. The precipitate produced from this 40% saturated solution was removed by centrifugation (16,000rpm for 20 minutes) and 137ml of supernatant liquid was obtained. Ammonium sulfate was added again to 80% saturation, and the thus produced precipitate was dissolved in 50ml of the above-mentioned 40mM tris-HCl buffer and was then dialyzed against same buffer containing 50mM NaCl.

### (Example 9)

High grade purification of reverse transcriptase: high grade purification was carried out using DEAE Bio-Gel A (made by BioRad [U. S. A]) and Affi-Gel Heparin column chromatography (made by BioRad). The dialysed sample of Example 8 was applied to a 30ml DEAE Bio-Gel A column equilibrated with 40mM tris-HCl (pH 8.0) (containing 0.1mM EDTA, 5mM MgCl₂, 0.1W/V% Triton X-100, 10mM 2-mercaptoethanol and 50mM NaCl). The eluted sample was then applied to a 30ml Affi-Gel Heparin column equilibrated with the above-mentioned buffer and was eluated with 150ml buffer comprising a sodium chloride gradient of from 50mM to 400mM. The result is shown in Fig. 4. Fractions 29 to 38 containing reverse transcriptase activity were pooled. The thus pooled reverse transcriptase fractions were dialyzed against 20mM sodium phosphate buffer (pH 6.8) (containing 0.1mM EDTA, 5mM MgCl₂, 0.1W/V% Triton X-100 and 10mM 2-mercaptoethanol) and were further purified by the use of hydroxylapatite column (KB column, made by Koken [Japan]) by high-performance liquid chromatography. More particularly, after adsorption of the above-mentioned dialyzed specimen onto the column, eluation was carried out with a linear gradient of sodium phosphate of 20 to 400mM, and fractions containing reverse transcriptase activity were pooled. Thus, purified reverse transcriptase was obtained. The thus obtained reverse transcriptase was confirmed, by the use of SDS-PAGE, to have a purity of over 95%. The yield was 31% relative to the crude extraction liquid.

### (Example 10)

Diagnosis of HIV-1 infection using purified reverse transcriptase: The purified reverse transcriptase (protein concentration 250»g/ml) prepared according to example 9 was electrophoresed on a polyacrylamide gel in accordance with example 2, and was blotted onto a nitrocellulose membrane. The membrane was then immersed in a 3W/V% gelatin solution for blocking. Subsequently, the presence of an antibody against the HIV-1 reverse transcriptase was investigated in the sera of human HIV-1 carriers (3 subjects) using the Western blot technique. Human T-cell leukemia virus (HTLV-1) carriers (5 subjects) and healthy adults (5 subjects) were similarly investigated. The result is shown in Table 1. The sera of all 3 HIV-1 carriers reacted to reverse transcriptase (66kd and 51kd). However, none of the sera of the HTLV-1 carriers (which belongs to same retrovirus family as HIV-1), nor the sera from the 5 healthy subjects did so. This suggests that it is possible to make a specific diagnosis of the presence of HIV-1 infection by using the purified HIV-1 reverse transcriptase prepared from Escherichia coli according to the present invention.

**Table 1**

| Diagnosis of HIV-1 infection by Western blotting, using the purified reverse transcriptase. | | |
|---|---|---|
| Subjects | | Reactivity * |
| human serum of HIV-1 carrier | 1 | + * * |
| | 2 | + |
| | 3 | + |
| human serum of HTLV-1 carrier | 1 | - |
| | 2 | - |
| | 3 | - |
| | 4 | - |
| | 5 | - |
| human serum of healthy adult | 1 | - |
| | 2 | - |
| | 3 | - |
| | 4 | - |
| | 5 | - |

| | | |
|---|---|---|
| * Specific immunoligical reaction against purified reverse transciptase. | | |
| * * Reactivity was measured by the Western blot technique. Shown are positive (+) and negative (-) reaction. | | |

### EFFECT OF THE INVENTION

(1) In the production method of the present invention, in which a very dangerous retrovirus itself is not used, a high safety is available from the point of view of biohazard under the production conditions, and the producing operation is easy.
(2) The method of the present invention provides a very high production yield of each of the enzymes produced as present by an amount of protein of from 1 to 10mg per liter of bacteria culture.
(3) According to the present invention , in spite of the retroviral protease, reverse transcriptase and integrase are expressed as a fusion protein with high expressing ability, various enzymes can be produced, not in the form of fusion protein, but in the form of single matured proteins which had been processed respectively. The method is thus more efficient and rational than that using the expression of single enzyme genes, and taking account of the effects (1) and (2) above, economical requiring a lower production cost.
(4) Since enzymes having a very high specificity relative to the substrate unique to retroviruses and enzymes as antigen to retroviruses are available at a low cost in a large quantity, the method of the present invention brings about a great progress in fundamental research on retrovirus infectious diseases such as AIDS, adult T cell leukemia, avian sarcoma or leukemia, and feline leukemia, and development of specific therapeutic drugs and preventive drugs having a high selective efficacy and diagnosis thereof, thus providing a boon to human health and promotion of stock breeding.
(5) Method for producing related to the present invention can be applied to development of the efficient and rational mass production of those gene products, for this method makes it possible to cause a mass expression and a after processing of various another genes contained in the said virus and retrotransposon, as well as of various retrovirus enzyme genes.

## Claims (Claims for the following Contracting State(s): AT, BE, CH, DE, FR, GB, GR, IT, LI, LU, NL, SE)

1. A method for preparing one or more retroviral proteins, said method comprising the steps of:
(i) constructing an expression vector comprising a cDNA sequence corresponding to one or more retroviral genes encoding at least one retroviral protein, linked in matching reading frame to a sequence corresponding to the entire lacZ gene or to a lacZ gene lacking the nucleotides coding for one or both of the two C-terminal amino acids, with the proviso that at least one of said retroviral genes encodes a protease;
(ii) introducing the said expression vector into cells of E.coli strain UT481 (FERM BP-2417);
(iii) culturing the transformed cells under conditions whereby the said genes are expressed as a fusion-protein which is subsequently processed in situ by the action of the said protease to form separate mature protein molecules, wherein where at least one other retroviral protein is expressed in addition to the protease, the transformed cells are cultured at about 25°C following induction.

2. A method as claimed in claim 1 wherein said retroviral proteins are selected from protease, reverse transcriptase, endonuclease and Gag proteins.

3. A method as claimed in claim 1 or claim 2, wherein said transformed recipient cells are cultured by a two-stage culturing method.

4. A method as claimed in any of claims 1 to 3, wherein the transformed recipient cells are pre-cultured for 1 to 24 hours at a temperature of from 10 to 40°C prior to induction.

5. A method as claimed in any one of claims 1 to 4, wherein said retroviral gene cDNA fragment is selected and prepared from E.coli UT481/pNLH402 (FERM BP-2417) and plasmid pSRA2.

6. A method as claimed in any one of claims 1 to 5, wherein said retroviral gene cDNA fragment is inserted in one of the cloning sites at the 3'end of the lacZ gene of a plasmid pUR290 series vector.

7. An expression vector comprising a cDNA sequence corresponding to one or more retroviral genes encoding at least one retroviral protein, linked in matching reading frame to a sequence corresponding to the entire lacZ gene or to a lacZ gene lacking the nucleotides coding for one or both of the two C-terminal amino acids, with the proviso that at least one of said retroviral genes encodes protease.

## Claims (Claims for the following Contracting State(s): ES)

1. A method for preparing one or more retroviral proteins, said method comprising the steps of:
(i) constructing an expression vector comprising a cDNA sequence corresponding to one or more retroviral genes encoding at least one retroviral protein, linked in matching reading frame to a sequence corresponding to the entire lacZ gene or to a lacZ gene lacking nucleotide coding for one or both of the two C-terminal amino acids, with the proviso that at least one of said retroviral genes encodes a protease;
(ii) introducing the said expression vector into cells of E.coli strain UT481 (FERM BP-2417);
(iii) culturing the transformed cells under conditions whereby the said genes are expressed as a fusion-protein which is subsequently processed in situ by the action of the said protease to form separate mature protein molecules, wherein where at least one other retroviral protein is expressed in addition to the protease, the transformed cells are cultured at about 25°C following induction.

2. A method as claimed in claim 1 wherein said retroviral proteins are selected from protease, reverse transcriptase, endonuclease and Gag proteins.

3. A method as claimed in claim 1 or claim 2, wherein said transformed recipient cells are cultured by a two-stage culturing method.

4. A method as claimed in any of claims 1 to 3, wherein the transformed recipient cells are pre-cultured for 1 to 24 hours at a temperature of from 10 to 40°C prior to induction.

5. A method as claimed in any one of claims 1 to 4, wherein said retroviral gene cDNA fragment is selected and prepared from E.coli UT481/pNLH402 (FERM BP-2417) and plasmid pSRA2.

6. A method as claimed in any one of claims 1 to 5, wherein said retroviral gene cDNA fragment is inserted in one of the cloning sites at the 3'end of the lacZ gene of plasmid pUR290 series vector.

## Patentansprüche (Patentansprüche für folgende(n) Vertragsstaat(en): AT, BE, CH, DE, FR, GB, GR, IT, LI, LU, NL, SE)

1. Verfahren zur Herstellung von einem oder mehreren retroviralen Proteinen, umfassend folgende Schritte:
(i) die Herstellung eines Expressionsvektors, der eine cDNA-Sequenz umfaßt, die einem oder mehreren retroviralen, für wenigstens ein retrovirales Protein codierenden Genen entspricht, die unter Wahrung des Leserasters mit einer Sequenz verbunden ist, die dem gesamten lacZ-Gen oder einem lacZ-Gen, dem die für eine oder beide der Zwei C-terminalen Aminosäuren codierenden Nucleotide fehlen, entspricht, mit der Maßgabe, daß wenigstens eines der retroviralen Gene für eine Protease codiert;
(ii) die Einführung des Expressionsvektors in Zellen des E.coli-Stammes UT481 (FERM BP-2417);
(iii) die Kultivierung der transformierten Zellen unter Bedingungen, bei denen die Gene als Fusionsprotein exprimiert werden, das nachfolgend in situ durch die Wirkung der Protease prozessiert wird, wodurch getrennte, reife Proteinmoleküle gebildet werden, und wobei, wenn wenigstens ein weiteres retrovirales Protein zusätzlich zu der Protease exprimiert wird, die transformierten Zellen nach der Induktion bei etwa 25°C kultiviert werden.

2. Verfahren gemäß Anspruch 1, wobei die retroviralen Proteine ausgewählt sind unter einer Protease, Reverser Transcriptase, Endonuclease und Gag-Proteinen.

3. Verfahren gemäß Anspruch 1 oder Anspruch 2, wobei die transformierten Rezipientenzellen durch ein zweistufiges Kultivierungsverfahren kultiviert werden.

4. Verfahren gemäß einem der Ansprüche 1 bis 3, wobei die transformierten Rezipientenzellen vor der Induktion 1 bis 24 Stunden bei einer Temperatur von 10 bis 40°C in Vorkultur gehalten werden.

5. Verfahren gemäß einem der Ansprüche 1 bis 4, wobei das cDNA-Fragment des retroviralen Gens ausgewählt und hergestellt wird aus E.coli UT481/pNLH402 (FERM BP-2417) und dem Plasmid pSRA2.

6. Verfahren gemäß einem der Ansprüche 1 bis 5, wobei das cDNA-Fragment des retroviralen Gens in eine der Clonierungsstellen am 3'-Ende des lacZ-Gens eines Plasmid-Vektors der Serie pUR290 insertiert wird.

7. Expressionsvektor, umfassend eine cDNA-Sequenz, die einem oder mehreren retroviralen, für wenigstens ein retrovirales Protein codierenden Genen entspricht, die unter Wahrung des Leserasters mit einer Sequenz verbunden ist, die dem gesamten lacZ-Gen oder einem lacZ-Gen, dem die für eine oder beide der zwei C-terminalen Aminosäuren codierenden Nucleotide fehlen, entspricht, mit der Maßgabe, daß wenigstens eines der retroviralen Gene für eine Protease codiert.

## Patentansprüche (Patentansprüche für folgende(n) Vertragsstaat(en): ES)

1. Verfahren zur Herstellung von einem oder mehreren retroviralen Proteinen, umfassend folgende Schritte:
(i) die Herstellung eines Expressionsvektors, der eine cDNA-Sequenz umfaßt, die einem oder mehreren retroviralen, für wenigstens ein retrovirales Protein codierenden Genen entspricht, die unter Wahrung des Leserasters mit einer Sequenz verbunden ist, die dem gesamten lacZ-Gen oder einem lacZ-Gen, dem die für eine oder beide der zwei C-terminalen Aminosäuren codierenden Nucleotide fehlen, entspricht, mit der Maßgabe, daß wenigstens eines der retroviralen Gene für eine Protease codiert;
(ii) die Einführung des Expressionsvektors in Zellen des E.coli-Stammes UT481 (FERM BP-2417);
(iii) die Kultivierung der transformierten Zellen unter Bedingungen, bei denen die Gene als Fusionsprotein exprimiert werden, das nachfolgend in situ durch die Wirkung der Protease prozessiert wird, wodurch getrennte, reife Proteinmoleküle gebildet werden, und wobei, wenn wenigstens ein weiteres retrovirales Protein zusätzlich zu der Protease exprimiert wird, die transformierten Zellen nach der Induktion bei etwa 25°C kultiviert werden.

2. Verfahren gemäß Anspruch 1, wobei die retroviralen Proteine ausgewählt sind unter einer Protease, Reverser Transcriptase, Endonuclease und Gag-Proteinen.

3. Verfahren gemäß Anspruch 1 oder Anspruch 2, wobei die transformierten Rezipientenzellen durch ein zweistufiges Kultivierungsverfahren kultiviert werden.

4. Verfahren gemäß einem der Ansprüche 1 bis 3, wobei die transformierten Rezipientenzellen vor der Induktion 1 bis 24 Stunden bei einer Temperatur von 10 bis 40°C in Vorkultur gehalten werden.

5. Verfahren gemäß einem der Ansprüche 1 bis 4, wobei das cDNA-Fragment des retroviralen Gens ausgewählt und hergestellt wird aus E.coli UT481/pNLH402 (FERM BP-2417) und dem Plasmid pSRA2.

6. Verfahren gemäß einem der Ansprüche 1 bis 5, wobei das cDNA-Fragment des retroviralen Gens in eine der Clonierungsstellen am 3'-Ende des lacZ-Gens eines Plasmid-Vektors der Serie pUR290 insertiert wird.

## Revendications (Revendications pour l'(les) Etat(s) contractant(s) suivant(s): AT, BE, CH, LI, DE, FR, GB, GR, IT, LU, NL, SE)

1. Procédé de préparation d'une ou plusieurs protéines rétrovirales, caractérisé en ce qu'il comprend les étapes suivantes :
(i) construction d'un vecteur d'expression comprenant une séquence d'ADNc correspondant à un ou plusieurs gènes rétroviraux encodant au moins une protéine rétrovirale, liée en cadre de lecture assorti ou apparié à une séquence correspondant au gène de lacZ entier ou un gène de lacZ dépourvu des nucléotides codant pour l'un des ou les deux aminoacides C-terminaux, avec la condition qu'au moins l'un desdits gènes rétroviraux encode une protéase;
(ii) introduction dudit vecteur d'expression dans des cellules de la souche de E. coli UT481 (FERM BP-2417);
(iii) culture des cellules transformées dans des conditions selon lesquelles lesdits gènes sont exprimés sous forme d'une protéine de fusion qui est subséquemment traitée in situ par l'action de ladite protéase pour former des molécules de protéines matures séparées, opération où au moins une autre protéine rétrovirale est exprimée en plus de la protéase, les cellules transformées étant cultivées à environ 25°C après l'induction.

2. Procédé suivant la revendication 1, caractérisé en ce que lesdites protéines rétrovirales sont choisies parmi des protéines de protéase, de transcriptase inverse, d'endonucléase et de gag.

3. Procédé suivant la revendication 1 ou la revendication 2, caractérisé en ce que lesdites cellules receveuses transformées sont cultivées selon un procédé de culture à deux stades.

4. Procédé suivant l'une quelconque des revendications 1 à 3, caractérisé en ce que les cellules receveuses transformées sont soumises à une culture préalable ou préculture de 1 à 24 heures à une température de 10 à 40°C avant l'induction.

5. Procédé suivant l'une quelconque des revendications 1 à 4, caractérisé en ce que le fragment d'ADNc de gène rétroviral susmentionné est choisi parmi et préparé à partir d'E. coli UT481/pNLH402 (FERM BP-2417) et du plasmide pSRA2.

6. Procédé suivant l'une quelconque des revendications 1 à 5, caractérisé en ce que le fragment d'ADNc de gène rétroviral susmentionné est inséré dans l'un des sites de clonage à l'extrémité 3' du gène de lacZ d'un vecteur de série du plasmide pUR290.

7. Vecteur d'expression comprenant une séquence d'ADNc correspondant à un ou plusieurs gènes encodant au moins une protéine rétrovirale, liée en un cadre de lecture assorti ou apparié à une séquence correspondant au gène de lacZ entier ou à un gène de lacZ dépourvu des nucléotides codant pour l'un des ou les deux aminoacides C-terminaux, avec la condition qu'au moins l'un des gènes rétroviraux précités encode la protéase.

## Revendications (Revendications pour l'(les) Etat(s) contractant(s) suivant(s): ES)

1. Procédé de préparation d'une ou plusieurs protéines rétrovirales, caractérisé en ce qu'il comprend les étapes suivantes :
(i) construction d'un vecteur d'expression comprenant une séquence d'ADNc correspondant à un ou plusieurs gènes rétroviraux encodant au moins une protéine rétrovirale, liée en cadre de lecture assorti ou apparié à une séquence correspondant au gène de lacZ entier ou un gène de lacZ dépourvu des nucléotides codant pour l'un des ou les deux amino-acides C-terminaux, avec la condition qu'au moins l'un desdits gènes rétroviraux encode une protéase;
(ii) introduction dudit vecteur d'expression dans des cellules de la souche de E. coli UT481 (FERM BP-2417);
(iii) culture des cellules transformées dans des conditions selon lesquelles lesdits gènes sont exprimés sous forme d'une protéine de fusion qui est subséquemment traitée in situ par l'action de ladite protéase pour former des molécules de protéines matures séparées, opération où au moins une autre protéine rétrovirale est exprimée en plus de la protéase, les cellules transformées étant cultivées à environ 25°C après l'induction.

2. Procédé suivant la revendication 1, caractérisé en ce que lesdites protéines rétrovirales sont choisies parmi des protéines de protéase, de transcriptase inverse, d'endonucléase et de gag.

3. Procédé suivant la revendication 1 ou la revendication 2, caractérisé en ce que lesdites cellules receveuses transformées sont cultivées selon un procédé de culture à deux stades.

4. Procédé suivant l'une quelconque des revendications 1 à 3, caractérisé en ce que les cellules receveuses transformées sont soumises à une culture préalable ou préculture de 1 à 24 heures à une température de 10 à 40°C avant l'induction.

5. Procédé suivant l'une quelconque des revendications 1 à 4, caractérisé en ce que le fragment d'ADNc de gène rétroviral susmentionné est choisi parmi et préparé à partir d'E. coli UT481/pNLH402 (FERM BP-2417) et du plasmide pSRA2.

6. Procédé suivant l'une quelconque des revendications 1 à 5, caractérisé en ce que le fragment d'ADNc de gène rétroviral susmentionné est inséré dans l'un des sites de clonage à l'extrémité 3' du gène de lacZ d'un vecteur de série du plasmide pUR290.
